# NEW EUROPEAN PATENT SPECIFICATION

(11) **EP 0 967 892 B2**
(45) Date of publication and mention of the opposition decision: **10.10.2007**
(45) Mention of the grant of the patent: 29.12.2004
(21) Application number: 97918069.2
(22) Date of filing: 18.04.1997
(51) Int. Cl.: A23L 2/66

(54) **ADDITIVE FOR USE IN ANY ENERGY SUPPLEMENTATION, USE OF A PROTEIN HYDROLYSATE TO THE PREPARATION OF ENERGY SUPPLEMENTATON AND AN ENERGY SUPPLEMENT CONTAINING SUCH AN ADDITIVE**
ZUSATZ ZU EINEM DIÄTETISCHEN ENERGIETRÄGER, VERWENDUNG EINES PROTEINHYDROLYSATES ZUR HERSTELLUNG EINES ENERGIETRÄGERS UND EIN ENERGIETRÄGER, DER EINEN SOLCHEN ZUSATZ ENTHÄLT
ADDITIF UTILISE DANS TOUT TYPE DE COMPLEMENTS ENERGETIQUES, UTILISATION D'UN HYDROLYSAT DE PROTEINES DANS LA PREPARATION D'UN COMPLEMENT ENERGETIQUE, ET COMPLEMENT ENERGETIQUE COMPRENANT CET ADDITIF

(30) Priority: 18.04.1996 DK 45796
(43) Date of publication of application: 05.01.2000
(73) Proprietor: Arla Foods Amba, 8260 Viby J (DK)
(72) Inventor: BERTHELSEN, Jorn, Havskov, DK-3000 Helsingor (DK); FROKJAER, Sven, DK-2840 Holte (DK); SALTIN, Bengt, DK-4540 Faarevejle (DK)
(74) Representative: Wetke, Ellen
(86) International application number: PCT/DK1997/000177
(87) International publication number: WO 1997/039641

(56) References cited:
- EP-A- 0 629 350
- WO-A-92/15696
- WO-A-92/15697
- US-A- 4 107 334
- Ziegler et al. "Efficiency of enteral nitrogen support in surgical patients:small peptides v. non-degraded products" Gut, 1990, 31,p. 1277-1283
- Zawadzki et al. "Carbohydrate-protein complex increases the rate of muscle glycogen storage after exercise" p.1854-1859
- Adler-Nissen "Enzymic Hydrolisis of Food Proteins", Elsevier Applied Science Publishers 1986, pp. 9-15
- Gannon et al, Metabolism, Vol. 36, N°6, 1987, pp. 595-600

## Description

The present invention relates to the hitherto unknown use of a protein hydrolysate as an additive to an energy supplementation or metabolic nutrient to increase the glycogen level for a person in need of an increased glycogen level, to rise the insulin secretion and/or to reduce muscle break-down during metabolic or physical stress.

WO 92/15696 and WO 92/15697 disclose methods of preparation of protein hydrolysates having a degree of hydrolysis (DH) between 15 and 35. It is proposed to use such protein hydrolysates in soft drinks instead of the parent proteins. It is not proposed to use the protein hydrolysates to increase glycogen level, rise insulin secretion or to reduce muscle break-down.

The invention can be used in an energy supplementation or metabolic nutrient in the form of a beverage or other nutrient preparation for an athlete or another person in need of increased glycogen level and an additive for such a beverage or other nutrient for oral intake.

Athletes consume energy, primarily glycogen (carbohydrate) and later on (after 40 minutes) fat. Carbohydrate is a fast and light energy source. Consequently, carbohydrates are taken in together with liquid. In order that carbohydrates can be utilised they must pass from the blood into muscles. This transport requires insulin.

As it is, intake of water + carbohydrate increases insulin secretion moderately.

According to the invention a study has surprisingly shown that the combination water + carbohydrates + special protein hydrolysate increases insulin secretion dramatically.

This is interesting because insulin is considered an anabolic (building) hormone, and is necessary both for optimum energy uptake in muscles and brain and for uptake in (transport to) the muscles of amino acids, and is also necessary when rebuilding the spent glycogen.

This means that
1) the special protein hydrolysate of the invention + carbohydrate + water increases insulin secretion and consequently the chance of supplying more energy to muscles when exercising than carbohydrate + water or carbohydrate + water + intact protein.
2) after the metabolic stress, intake of the energy supplement of the invention in the form of a beverage or other nutrient entails faster rebuilding of glycogen depots and faster rebuilding of degraded muscular proteins.

The additive is characterized in that it is a protein hydrolysate having a degree of hydrolysis (DH) of 1-50, preferably 15-30 and especially about 25, which protein hydrolysate after oral intake rises the insulin secretion to a level above what is achieved by intake of the intact parent protein. Preferably the protein hydrolysate does not increase the gastric emptying, generally gives persons less satiety, increases the glycogen synthesis or rebuilding after depletion and reduces muscle break-down during metabolic stress, due to increased influx of amino acids into the muscle compared to what is achieved by intake of the intact parent protein.

Studies have surprisingly shown that both animal and vegetable protein is useful in the invention, which consequently comprises both types.

The protein hydrolysate can. thus be of animal or vegetable origin. Useful protein hydrolysates are whey protein hydrolysates, casein protein hydrolysates, soy protein hydrolysates, wheat protein hydrolysates or pea protein hydrolysates.

The use of a protein hydrolysate to the preparation of an energy supplement or metabolic nutrient is as defined in claims 4 to 9 and the energy supplementation as defined in the claims 10 to 11.

The proteins useful in the invention can be obtained by enzymatic or non-enzymatic hydrolysis e.g. by enzymatic hydrolysis as described in the international patent application WO 92/21248.

An additive can be used in a beverage or other nutrient product for athletes to reestablish liquid and salt balance as well as glycogen levels in the body before, under or after sport or other physical work. The beverage is preferable an isotonic fluid. The nutrient can be liquid as well as solid or be a mixture of liquids and solids, such as a paste. It can also contain sweeteners, flavours and other ingredients normally contained in beverages of this kind.

The energy supplementation contains a protein hydrolysate, a carbohydrate and optionally water or it is taken together with water.

The carbohydrate can be glucose, sucrose, maltose or maltodextrines or mixtures thereof.

The following is an example of a useful energy supplementation in the form of a beverage.

| **GENERAL EXAMPLE** | |
|---|---|
| Ingredients, pr. liter: | |
| Protein hydrolysate | 30 g ± 15 g |
| Carbohydrate | 70 g ± 30 g |
| Sodium | 25 mM ± 10 mM |
| Potassium | 3 mM ± 2 mM |
| Chloride | 12 mM ± 6 mM |

Carbohydrate can be glucose, sucrose, maltose and/or maltodextrines.

Trace amounts of other mineral can be present.

| **SPECIFIC EXAMPLE** | |
|---|---|
| Lacprodan DI-3065 | 3.75% |
| SPG 30 (DE 28-32) | 3.50% |
| SPG 20 (DE 20-23) | 3.50% |
| NaCl | 0.07% |
| NaOH | 0.03% |
| Aspartam | 0.03% |
| Flavour | 0.40% |
| Malic Acid | 0.45% |
| Water | 88.27% |

### EXPERIMENTS

The following experiments have been carried out.

### RATE OF GASTRIC EMPTYING AND PLASMA INSULIN RESPONSE TO PROTEIN HYDROLYSATE SOLUTIONS.

The purpose of the present study was to determine the rate of gastric emptying of four solutions containing different quantities of proteins and protein hydrolysates (approx. 4 amino acid residuals).

Six healthy human subjects (three males and three females; 22±2.1 years; 73.2±7.8 kg weight and 177.3±10.00 cm height) participated in this study, which was approved by the local ethics committee. Test meals (600 ml) were administered through a nasogastric tube, 30 minutes after a gastric washout. Four different meals were studied in random order, over a period of 4 weeks. Solution A (control) was composed of glucose (0.025 g ml⁻¹) and NaCl (0.9%). The three other solutions contained similar quantities of protein (0.25 g (kg body mass)⁻¹) and glucose (0.025 g ml⁻¹). Solutions were composed of: B, a pea protein hydrolysate and NaCl; C, a whey protein hydrolysate and NaCl; and D, milk powder. Solutions B and C were matched for osmolality, caloric density, and nitrogen contents, but contained different amounts of essential amino acids (35.5 and 45.1%, respectively) and non-essential amino acids (64.5 and 54.9%, respectively). Each meal was delivered at 37°C, after adjusting it to a pH of approx. 7. During the 3 h that each trial lasted, blood samples were obtained every 20 min. for the assessment of the plasmatic concentrations of glucose and insulin. Gastric samples were taken every 10 min, to determine the volume remaining in the stomach using George's double-sampling technique, as applied by Beekers et al. (1988).

The rate of gastric emptying followed an exponential pattern in all cases (r = 0.86-0.99). Solution A was emptied faster than the other three solutions with a half-time of 9.4±1.2 min (p<0.05). However, D emptied at a slower rate (26.4±10.0 min, p<0.05, paired *t* test) than both B (16.3±5.4 min) and C (17.2±6.1 min). Despite important differences in amino acid composition, both protein hydrolysate solutions emptied at a similar rate. Caloric density was closely related to the half-time of gastric emptying (r = 0.96, p<0.05, n = 4) and with the rate at which calories were delivered to the duodenum (r = 0.99, p<0.001, n = 4). The insulin response was significantly different among solutions (p<0.001, ANOVA). The plasma insulin peak elicited by the C and B (80.3±50.7 and 84.8±34.9, µmol ml⁻¹, respectively) were approx. 2- and 4-fold greater than that produced by D (47.8±13.7 µmol ml⁻¹, p<0.05, paired *t* test) and A (28.7±18.0 µmol ml⁻¹ p<0.05, paired *t* test).

The results of the present study suggest that protein hydrolysate solutions differing in the amino acid composition, but matched for temperature, volume, osmolality, acidity and caloric density, are emptied at a similar rate. In addition, the ingestion of solutions combining glucose and protein hydrolysates produce a significant and unexpected increase in plasma insulin concentration.

## Claims

1. Use of a protein hydrolysate having a degree of hydrolysis (DH) of 1-50 for the manufacture of an energy supplementation or metabolic nutrient for a person in need of increased glycogen level.

2. Use of a protein hydrolysate having a degree of hydrolysis (DH) of 1-50 for the manufacture of an energy supplementation or metabolic nutrient to rise the insulin secretion to a level above what is achieved by intake of the intact parent protein.

3. Use of a protein hydrolysate having a degree of hydrolysis (DH) of 1-50 for the manufacture of an energy supplementation or metabolic nutrient to reduce muscle break-down during metabolic stress.

4. Use according to any of the claims 1-3, wherein the energy supplementation or metabolic nutrient further contains a carbohydrate.

5. Use according to claim 4, wherein the carbohydrate is glucose, sucrose, maltose or a maltodextrine or mixtures thereof.

6. Use according to any of the preceding claims, wherein the protein hydrolysate has a degree of hydrolysis of 15 - 30.

7. Use according to any of the preceding claims, wherein the protein hydrolysate has a degree of hydrolysis of about 25.

8. Use according to any of the preceding claims, wherein the protein hydrolysate is an animal protein hydrolysate or a vegetable protein hydrolysate.

9. Use according to claim 8, wherein the protein hydrolysate is an animal protein hydrolysate.

10. Use according to claim 8, wherein the protein hydrolysate is a whey protein hydrolysate, a casein protein hydrolysate, a soy protein hydrolysate, a wheat protein hydrolysate or a pea protein hydrolysate.

11. Use according to claim 8, wherein the protein hydrolysate is a whey protein hydrolysate.

## Patentansprüche

1. Verwendung eines Proteinhydrolysats mit einem Hydrolysegrad (DH) von 1-50 für die Herstellung eines Energiesupplements oder metabolischen Nährstoffs für eine Person, die einen erhöhten Glycogenspiegel benötigt.

2. Verwendung eines Proteinhydrolysats mit einem Hydrolysegrad (DH) von 1-50 für die Herstellung eines Energiesupplements oder metabolischen Nährstoffs zum Steigern der Insulinsekretion auf einen Wert, der höher ist als der Wert, der durch die Einnahme des intakten Ausgangsproteins erzielt wird.

3. Verwendung eines Proteinhydrolysats mit einem Hydrolysegrad (DH) von 1-50 für die Herstellung eines Energiesupplements oder metabolischen Nährstoffs zum Verringern des Muskelabbaus während metabolischem Stress.

4. Verwendung nach einem der Ansprüche 1-3, wobei das Energiesupplement oder der metabolische Nährstoff außerdem ein Kohlenhydrat enthält.

5. Verwendung nach Anspruch 4, wobei das Kohlenhydrat Glucose, Sucrose, Maltose oder ein Maltodextrin oder Mischungen davon ist.

6. Verwendung nach einem der vorangehenden Ansprüche, wobei das Proteinhydrolysat einen Hydrolysegrad von 15-30 aufweist.

7. Verwendung nach einem der vorangehenden Ansprüche, wobei das Proteinhydrolysat einen Hydrolysegrad von ungefähr 25 aufweist.

8. Verwendung nach einem der vorangehenden Ansprüche, wobei das Proteinhydrolysat ein Tierproteinhydrolysat oder ein Pflanzenproteinhydrolysat ist.

9. Verwendung nach Anspruch 8, wobei das Proteinhydrolysat ein Tierproteinhydrolysat ist.

10. Verwendung nach Anspruch 8, wobei das Proteinhydrolysat ein Molkenproteinhydrolysat, ein Kaseinproteinhydrolysat, ein Sojaproteinhydrolysat, ein Weizenproteinhydrolysat oder ein Erbsenproteinhydrolysat ist.

11. Verwendung nach Anspruch 8, wobei das Proteinhydrolysat ein Molkenproteinhydrolysat ist.

## Revendications

1. Utilisation d'un hydrolysat de protéine, ayant un degré d'hydrolyse (DH) de 1-50, pour la fabrication d'un complément énergétique ou d'un substrat métabolique pour une personne nécessitant un niveau de glycogène accru.

2. Utilisation d'un hydrolysat de protéine, ayant un degré d'hydrolyse (DH) de 1-50, pour la fabrication d'un complément énergétique ou d'un substrat métabolique pour porter la sécrétion d'insuline à niveau supérieur à celui atteint par un apport de protéine parente intacte.

3. Utilisation d'un hydrolysat de protéine, ayant un degré d'hydrolyse (DH) de 1-50, pour la fabrication d'un complément énergétique ou d'un substrat métabolique, pour réduire une dégradation musculaire au cours d'une agression métabolique.

4. Utilisation selon l'une quelconque des revendications 1 à 3, dans laquelle le complément énergétique ou le substrat métabolique contient, en outre, un hydrate de carbone.

5. Utilisation selon la revendication 4, dans laquelle l'hydrate de carbone est le glucose, le saccharose, le maltose ou une maltodextrine, ou leurs mélanges.

6. Utilisation selon l'une quelconque des revendications précédentes, dans laquelle l'hydrolysat de protéine a un degré d'hydrolyse de 15-30.

7. Utilisation selon l'une quelconque des revendications précédentes, dans laquelle l'hydrolysat de protéine a un degré d'hydrolyse d'environ 25.

8. Utilisation selon l'une quelconque des revendications précédentes, dans laquelle l'hydrolysat de protéine est un hydrolysat de protéine animale ou un hydrolysat de protéine végétale.

9. Utilisation selon la revendication 8, dans laquelle l'hydrolysat de protéine est un hydrolysat de protéine animale.

10. Utilisation selon la revendication 8, dans laquelle l'hydrolysat de protéine est un hydrolysat de protéine de petit-lait, un hydrolysat de protéine de caséine, un hydrolysat de protéine de soja, un hydrolysat de protéine de blé ou un hydrolysat de protéine de pois.

11. Utilisation selon la revendication 8, dans laquelle l'hydrolysat de protéine est un hydrolysat de protéine de petit-lait.
